# EUROPÄISCHE PATENTSCHRIFT

(11) **EP 0 628 552 B1**
(45) Veröffentlichungstag und Bekanntmachung des Hinweises auf die Patenterteilung: **27.08.1997**
(21) Anmeldenummer: 94105548.5
(22) Anmeldetag: 11.04.1994
(51) Int. Cl.: C07D 307/33, C07B 41/06

(54) **Verfahren zur katalytischen Dehydrierung von Butandiol-1,4**
Process for the catalytic dehydration of 1,4-butanediol
Procédé pour la deshydration catalytique du butanediol-1,4

(30) Priorität: 11.06.1993 DE 4319456
(43) Veröffentlichungstag der Anmeldung: 14.12.1994
(73) Patentinhaber: HÜLS AKTIENGESELLSCHAFT, 45764 Marl (DE)
(72) Erfinder: Sigg, Reinhard, Dr., D-45772 Marl (DE); Regner, Hans, Dr., D-45770 Marl (DE)

(56) Entgegenhaltungen:
- WO-A-92/00973
- CHEMICAL ABSTRACTS, vol. 106, no. 17, 27. April 1987, Columbus, Ohio, US; abstract no. 138244w, Seite 672 ; & JP-A-61 246 173 (IDEMITSU PETROLEUM CO., LTD.)
- Patai,The chemistry of ethers,crown ethers,hydroxy groups and their sulphur analogues,Supplement E,Part 2,Chichester:John Wiley & Sons,1980,721-760

## Beschreibung

Die Erfindung betrifft ein Verfahren zur katalytischen Dehydrierung von Butandiol-1,4 an einem Kupfer und Chrom enthaltenden Katalysator.

Bei der katalytischen Dehydrierung von Diolen bilden sich hauptsächlich Aldehyde und Lactone, die unter anderem als Riechstoffe Verwendung finden, als Lösemittel dienen und darüber hinaus Vorprodukte für pharmazeutische Präparate und Polymere darstellen.

Die katalytische Dehydrierung von Alkoholen oder Diolen an Kupfer und Chrom enthaltenden Katalysatoren ist allgemein bekannt. So wird die Herstellung von Lactonen durch Dehydrierung an Kupferoxid in der Flüssigphase beschrieben (siehe B. Berthon et. al., Tetrahedron Letters 22 (41) 4073-6 (1968). Auch aus Firmenprospekten, z. B. der Firma Südchemie, Mallinckrodt und Harshaw (Engelhard) ist die Verwendung von Cu/Cr-Katalysatoren mit und ohne BaO-Zusatz für die Dehydrierung von Alkoholen bekannt. JP-OS 86/2461 73 lehrt die Herstellung von γ-Butyrolacton aus Butandiol an Cu/Cr/Mn- oder Cu/Cr/Zn-Katalysatoren. Zur Erhöhung der Lebensdauer des Katalysators wird Wasserstoff zugegeben. Gemäß WO 92/00 973 werden Diole dehydriert, wobei in der Flüssigphase ein feinteiliger, suspendierter Kupferoxid- oder Kupfer/Chrom-Katalysator verwendet wird. Die Dehydrierung kann gemäß dem Stand der Technik in der Flüssigphase an Festbettkatalysatoren oder in Suspension sowie in der Gasphase an Festbettkatalysatoren durchgeführt werden.

Der Nachteil bei der Gasphasendehydrierung liegt in der relativ schnellen Desaktivierung des Katalysators. Außerdem wird bei den erforderlichen, relativ hohen Reaktionstemperaturen die Selektivität erniedrigt. Auch die Versuche, durch Zugabe von zusätzlichem Wasserstoff die Katalysatorlebensdauer zu erhöhen und die meistens hohen Siedetemperaturen der Diole durch Inertgasverdünnung zu senken und somit die Selektivität zu erhöhen, führen zwar zu geringfügigen Verbesserungen, verschlechtern aber gleichzeitig die Wirtschaftlichkeit des Verfahrens.

Die Flüssigphasendehydrierung leidet an zu niedrigen Selektivitäten und zu geringen Umsätzen.

Der Erfindung liegt die Aufgabe zugrunde, ein wirtschaftliches Verfahren zur katalytischen Dehydrierung von Butandiol-1,4 zu entwickeln, das sich durch hohe Selektivität und hohen Umsatz auszeichnet. Außerdem soll die Lebensdauer des Kupfer und Chrom enthaltenden Dehydrierungskatalysators verlängert werden.

Es wurde nun überraschenderweise gefunden, daß eine sehr hohe Selektivität bei praktisch vollständigem Umsatz erzielt werden kann, wenn die katalytische Dehydrierung in zwei Stufen durchgeführt wird, indem man das Butandiol-1,4 zunächst in der Flüssigphase und das so erhaltene Reaktionsgemisch anschließend in der Gasphase über den Katalysator leitet. Es ist sehr überraschend, daß bei dieser Reaktionsführung die Kupfer und Chrom enthaltenden Katalysatoren eine hohe Lebensdauer bewahren, obwohl in der zweiten Stufe ein Gasphasenprozeß abläuft und kein zusätzlicher Wasserstoff zugeführt wird.

Gegenstand der vorliegenden Erfindung ist daher ein Verfahren zur Dehydrierung von Butandiol-1,4 an einem Kupfer und Chrom enthaltenden Katalysator,
das dadurch gekennzeichnet ist,
daß in einem 2-Stufen-Verfahren Butandiol-1,4 zunächst in der Flüssigphase über den Katalysator geleitet und das so erhaltene Reaktionsgemisch anschließend in der Gasphase über den Katalysator geleitet wird.

Vorzugsweise wird die Dehydrierung bei Normaldruck oder unter geringem Überdruck durchgeführt. Hierbei sollte der Überdruck geeigneterweise nicht größer als 3 bar abs. sein.

Die erfindungsgemäße Dehydrierung wird in 2 Stufen zunächst in der 1. Stufe in der Flüssigphase und in der 2. Stufe in der Gasphase als "finishing-Dehydrierung" ausgeführt. Bei der "finishing-Dehydrierung" in der Gasphase in der 2. Stufe werden die letzten Reste des Einsatzstoffes umgesetzt. So kann geeigneterweise der Umsatz an Butandiol-1,4 in der Flüssigphase in der 1. Stufe ca. 70 bis 95 %, bevorzugt ca. 90 %, betragen. Die erfindungsgemäße Dehydrierung erfolgt sowohl in der Flüssigphase als auch in der Gasphase an bekannten Kupfer und Chrom enthaltenden Katalysatoren, wie sie samt ihren Herstellverfahren im oben zitierten Stand der Technik beschrieben sind, wie z. B. in der JP-OS 86/24 61 73. Es können auch alle handelsüblichen Kupfer und Chrom enthaltenden Katalysatoren, wie z. B. Kupferchromit-Katalysatoren in stückiger Form, eingesetzt werden.

Die erfindungsgemäße Dehydrierung sollte sowohl in der Flüssigphase wie auch in der Gasphase geeigneterweise unter schonenden Temperaturbedingungen erfolgen. Bei den einzustellenden Dehydrierungstemperaturen ist der Siedepunkt des eingesetzten Butandiols-1,4 zu beachten.

Die aus der 1. Stufe in der Flüssigphase entnommene Reaktionsmischung wird geeigneterweise nach weiterer Aufheizung vollständig in die zweite Stufe der Gasphasendehydrierung geleitet.

Die Dehydrierung in der 2. Stufe in der Gasphase erfolgt ohne die Zugabe von zusätzlichem Wasserstoff.

Das erfindungsgemäße Verfahren läßt sich in der Weise ausführen, daß die Dehydrierung in der Flüssigphase in einem ersten Reaktor und die "finishing-Dehydrierung" in der Gasphase dann in einem zweiten Reaktor erfolgt.

Die erfindungsgemäße Dehydrierung von Butandiol-1,4 kann unter schonenden Bedingungen bei Temperaturen von 150 bis 220 °C in der Flüssigphase und dann in der Gasphase bei Temperaturen von 180 bis 240 °C durchgeführt werden.

Also kann vorzugsweise in einem 2-Stufen-Verfahren Butandiol-1,4 zunächst in der Flüssigphase bei Temperaturen von 150 bis 220 °C über den Katalysator geleitet und das so erhaltene Reaktionsgemisch anschließend in der Gasphase bei Temperaturen von 180 bis 240 °C über den Katalysator geleitet werden.

Die Erfindung wird durch die folgenden Beispiele näher erläutert:

### Beispiel 1

An einem kommerziellen Cu/Cr-Katalysator (Mallinckrodt E 406 Tu 1/8 inch) mit 42 Gew.-% Cu, 40 Gew.-% Cr, der mit 8 Gew.-% BaO stabilisiert ist, wird auf 190 °C vorerhitztes Butandiol-1,4 in der 1. Stufe in einem Festbettreaktor bei einer Temperatur von 200 °C dehydriert. Der Festbettreaktor wird mit Wärmeträgeröl geheizt, und es wird so durch Wärmezufuhr eine isotherme Reaktionsführung der endothermen Reaktion (120 kjoule/kmol) gewährleistet. Das Butandiol-1,4 wird mit einer LHSV von 0,3 - 5 h⁻¹ von unten nach oben durch das Katalysatorbett geleitet. Am Kopf des Reaktors wird das Butyrolacton/Butandiol-1,4-Gemisch abgezogen und auf ca. 220 °C weitererhitzt und dann gasförmig von oben nach unten durch ein zweites Katalysatorbett, das ebenfalls mit Wärmeträgeröl beheizt wird, mit einer WHSV von 0,3 bis 5 h⁻¹ bei einer Temperatur von 230 °C geleitet.

Der Umsatz an Butandiol-1,4 ist größer als 99 % bei einer Selektivität zu γ-Butyrolacton von 97,8 %. Als Nebenprodukte entstehen geringe Mengen an Hydroxibutyraldehyd und cyclischem Acetal (Tetrahydrofuranbutandiolacetal).

### Beispiel 2

Unter den Reaktionsbedingungen aus Beispiel 1 wird die Dehydrierung von Butandiol-1,4 kontinuierlich über 2 000 h betrieben. Bei gleichbleibenden Reaktionstemperaturen von 190 bis 205 °C im ersten Katalysatorbett und 220 bis 235 °C im zweiten Katalysatorbett bleiben der Umsatz und die Selektivität mit den Werten aus Beispiel 1 praktisch konstant.

## Patentansprüche

1. Verfahren zur Dehydrierung von Butandiol-1,4 an einem Kupfer und Chrom enthaltenden Katalysator,
dadurch gekennzeichnet,
daß in einem 2-Stufen-Verfahren Butandiol-1,4 zunächst in der Flüssigphase über den Katalysator geleitet und das so erhaltene Reaktionsgemisch anschließend in der Gasphase über den Katalysator geleitet wird.

2. Verfahren nach Anspruch 1,
dadurch gekennzeichnet,
daß die Dehydrierung bei Normaldruck oder unter geringem Überdruck durchgeführt wird.

3. Verfahren nach den Ansprüchen 1 und 2,
dadurch gekennzeichnet,
daß in einem 2-Stufen-Verfahren Butandiol-1,4 zunächst in der Flüssigphase bei Temperaturen von 150 bis 220 °C über den Katalysator geleitet und das so erhaltene Reaktionsgemisch anschließend in der Gasphase bei Temperaturen von 180 bis 240 °C über den Katalysator geleitet wird.

## Claims

1. A process for the dehydrogenation of 1,4-butanediol on a catalyst containing copper and chromium, characterized in that in a 2-stage process first 1,4-butanediol is passed in the liquid phase over the catalyst, and subsequently the reaction mixture obtained in this way is passed in the gas phase over the catalyst.

2. A process according to claim 1, characterized in that the dehydrogenation is carried out under atmospheric pressure or slightly elevated pressure.

3. A process according to either of claims 1 and 2, characterized in that in a 2-stage process first 1,4-butanediol is passed in the liquid phase at temperatures from 150 to 220°C over the catalyst, and subsequently the reaction mixture obtained in this way is passed in the gas phase at temperatures from 180 to 240°C over the catalyst.

## Revendications

1. Procédé de déshydrogénation de butanediol-1,4 sur un catalyseur renfermant du cuivre et du chrome,
caractérisé en ce que
dans un procédé en deux étapes on conduit du butanediol-1,4 en premier lieu en phase liquide sur le catalyseur et ensuite le mélange réactionnel ainsi obtenu en phase gazeuse sur le catalyseur.

2. Procédé selon la revendication 1,
caractérisé en ce que
la déshydrogénation est effectuée à pression normale ou sous une légère surpression.

3. Procédé selon les revendications 1 et 2,
caractérisé en ce que
dans un procédé en deux étapes on conduit le butanediol-1,4 à des températures allant de 150° à 220°C en premier lieu en phase liquide sur le catalyseur et ensuite le mélange réactionnel ainsi obtenu en phase gazeuse à des températures allant de 180 à 240°C sur le catalyseur.
